# EUROPEAN PATENT SPECIFICATION

(11) **EP 0 383 453 B1**
(45) Date of publication and mention of the grant of the patent: **07.12.1994**
(21) Application number: 90300963.7
(22) Date of filing: 30.01.1990
(51) Int. Cl.: C12N 15/12, C12P 21/02

(54) **A reg protein**
Reg-Protein
Protéine Reg

(30) Priority: 30.01.1989 JP 22132/89
(43) Date of publication of application: 22.08.1990
(73) Proprietor: SHIONOGI SEIYAKU KABUSHIKI KAISHA trading under the name of SHIONOGI & CO. LTD., Osaka 541 (JP)
(72) Inventor: Okamoto, Hiroshi, Sendai-shi, Miyagi-ken (JP); Itoh, Takako, Takarazuka-shi, Hyogo-ken (JP); Teraoka, Hiroshi, Sakai-shi, Osaka (JP); Tsuzuki, Hiroshige, Tsuzuki-gun, Kyoto (JP); Yoshida, Nobuo, Nishinomiya-shi, Hyogo-ken (JP)
(74) Representative: Nash, David Allan

(56) References cited:
- EP-A- 0 303 233
- JOURNAL OF BIOLOGICAL CHEMISTRY, vol. 263, no. 5, 15th February 1988, pages2111-2114, The American Society for Biochemistry and Molecular Biology, Inc.,US; K. TERAZONO et al.: "A novel gene activated in regenerating islets"

## Description

The present invention relates to a new reg protein and a method for the production of the said protein.

Up until the present time, the generally accepted view has been that the B cells of the pancreatic islets lack regenerative capability and therefore that once these B cells are traumatized and necrotized, diabetes develops owing to insufficiency of insulin. However, in 1984, Okamoto et al. first succeeded in inducing the regeneration of B cells of pancreatic islets by continuous daily administration of poly(ADP-ribose) synthetase inhibitors, such as nicotinamide, to 90% pancreatectomized rats (Yonemura Y. et al., Diabetes 33, 401 (1984)). Moreover, some recent reports have indicated that a large amount of oral administration of nicotinamide prolongs remission periods in human insulin-dependent diabetes (Ph. Vague et al., Lancet, Vol. I, No. 8533, 619-620, 1987).

The techniques necessary for the differential hybridization used in the present invention have already been establish by Takasawa et al. (Takasawa, S. et al., Diabetes 35, 1178 (1986)). That is, a cDNA library prepared from rat insulinoma of B cells is screened by differential hybridization using as probes cDNAs prepared from poly(A)⁺ RNA derived from insulinoma and from normal pancreatic islets, thereby effectively isolating the gene which is specifically expressed in insulinoma.

The present inventors previously employed differential hybridization methods to discover the gene which is specifically expressed in regenerated rat pancreatic islet cells. Then, applying this gene as a probe to the screening of a cDNA library derived from the human pancreas, the present inventors discovered the human gene homologous with the aforementioned rat gene, and designated these as reg (regenerating gene) (J. Biol. Chem. 263, 2111-2114, 1988).

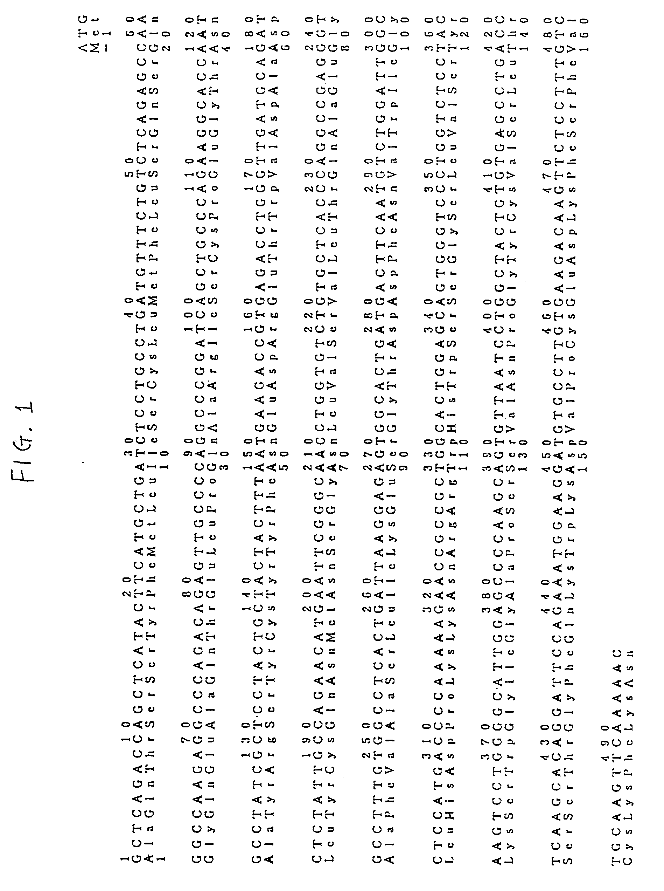

Furthermore, the present inventors introduced an expression vector possessing the gene shown in Figure 1 into yeast, cultivated the transformed yeast and induced the secretory production of the gene-encoding proteins by the transformants. The reg proteins obtained in this manner had the amino acid sequence shown in Figure 1 beginning with the alanine residue at the 1st position, the glycine residue at the 21st position, the glutamine residue at the 22nd position, the glutamic acid residue at the 23rd position, the alanine residue at the 24th position, the glutamine residue at the 25th position, the glutamine residue at the 30th position, the alanine residue at the 31st position or the isoleucine residue at 33rd position, reckoned from the amino terminus (Japanese Patent Application No. 63-195727).

Up until the present time, treatment of diabetes has been limited to symptomatic therapy comprising the process of administration of insulin; the oral administration of antidiabetic agents such as sulphonylurea, which at one time appeared promising, was found upon prolonged continuous administration to impede rather than promote insulin production by pancreatic B cells as well as inducing adverse effects such as coronary arteriosclerosis, etc.

According to this invention there is provided a reg protein having an amino acid sequence beginning with the glutamine residue at the 20th position from the amino terminus and terminating with the asparagine residue at the 165th position in Figure 1, or an amino acid sequence containing an additional methionine residue at the amino terminus of said amino acid sequence extending from the glutamine residue to the asparagine residue.

A method for producing a reg protein of this invention comprises the steps of, culturing host cells which have an expression vector containing a gene encoding the reg protein; and recovering said reg protein from the culture.

In a preferred embodiment, the host is yeast.

Thus, the invention described herein makes possible the objectives of: (1) providing a new reg protein that is effectively applicable as an agent for the treatment of diabetes; and (2) providing a gene encoding the aforementioned reg protein and a method for producing a reg protein employing the same.

For a better understanding of the invention and how the same can be carried into effect, reference will now be made, by way of example only, to the accompanying drawings, in which:
Figure 1 shows the cDNA base sequence of the human reg and the amino acid sequence deduced from the said base sequence.
Figure 2 shows the cDNA base sequence of the rat reg and the amino acid sequence deduced from the said base sequence.
Figure 3 is a schematic diagram showing the procedure for construction of an expression vector for the human reg.

### DESCRIPTION OF THE PREFERRED EMBODIMENTS

For the purpose of elucidating the mechanism of regeneration and proliferation of the B cells of pancreatic islets at the gene level, the present inventors investigated the genes which are specifically expressed in regenerating B cells in the pancreatic islets of rats, and thus discovered the rat reg (i.e., regenerating gene). The present inventors further investigated human reg using the rat reg as a probe, and thereby identified the human reg from a human pancreatic cDNA library.

The present inventors conducted intensive research for the purpose of the expression of the human reg in a microbial host in order to obtain human reg protein, and thereby succeeded in the expression of the said human reg in yeast. The human reg protein so obtained was purified and analysis confirmed that the amino acid composition and sequence of the product were indeed identical with those deduced from the DNA sequence.

The present inventors investigated the aforementioned human reg protein secreted by the yeast hosts in further detail, and thereby discovered a new reg protein having an amino acid sequence beginning with the glutamine residue at the 20th position from the amino terminus and terminating with the asparagine residue at the 165th position in Figure 1.

The rat reg used as a probe was prepared in the following manner.

### I. Isolation of regenerating pancreatic islets from rats

### (1) Preparation of 90% pancreatectomized rats

Male Wistar rats with body weight 200-300 g were laparotomized under ether anesthesia, the entire pancreas except for the parabiliary segment was excised (equivalent to 90% resection of the total pancreatic mass), and the abdominal wounds were closed. This 90% pancreatectomy technique was establish by Foglia, V. G. (Rev. Soc. Argent. Biol. 20, 21-37, 1944), and is now widely employed for the creation of animal models of insulin-dependent diabetes. The 90% pancreatectomized rats ordinarily reach a diabetic state within 1 month after the pancreatectomy.

### (2) Administration of nicotinamide

Single daily doses of nicotinamide (50 mg/ml saline) equivalent to 0.5 g per kilogram body weight were intraperitoneally administered to these rats from 7 days before to 3 months after 90% pancreatectomy. This administration of nicotinamide prevents or ameliorates the diabetic state in 90% pancreatectomized rats.

### (3) Isolation of regenerating pancreatic islets

The above-described procedures (1) and (2) induce regeneration of pancreatic islets in the pancreatic tissue remaining after resection, increasing the number of islets per unit volume of the pancreatic tissue as well as the size of individual islets. Most of the cells proliferating in this process are insulin-producing B cells, the numbers of glucagon-producing A cells and somatostatin-producing D cells remain unchanged (Yonemura, Y. et al., Diabetes 33, 401 (1984)). Three months after the pancreatectomy, the 90% pancreatectomized nicotinamide-treated rats were again laparotomized under Nembutal anesthesia, and the remaining portion of the pancreas was excised after being swollen with Hanks' solution. Next, Hanks' solution was added to the excised pancreases so that 5 ml of the solution was added per two pieces of the excised pancreases. Then, the pancreases were cut in pieces, after which 150 mg of bovine serum albumin (Sigma, Type V) and 40 mg of collagenase (Type IV, Wako Pure Chemical Industries Ltd.) were added, and the tissue was digested while agitating the mixture for 3-5 minutes at 37°C. After digestion, the digested tissue was suspended in 50 ml of Hanks' solution, then left standing undisturbed for 5 minutes to allow precipitation of the pancreatic islets. Next, the upper half layer of the liquid was discarded, 25 ml of fresh Hanks' solution was added and the digested tissue was resuspended. This suspending and standing operation was repeated 8 times, after which the regenerating pancreatic islets were collected under a stereoscopic microscope. In this manner, 1355 regenerating pancreatic islets were recovered from thirty-two 90% pancreatectomized nicotinamide-treated rats. The aforementioned procedure is essentially that of Okamoto et al. (H. Okamoto, Molecular and Cellular Biochemistry 37, 43-61, 1981).

### II. Preparation of complementary DNA (cDNA) library from rat regenerating pancreatic islets

### (1) Isolation of RNA

The isolated regenerating pancreatic islets were homogenized by ultrasonication in a solution containing 4 M guanidine thiocyanate, 10 mM sodium citrate (pH 7.0), 0.5% sodium N-lauroylsarcosinate, 0.1 M 2-mercaptoethanol and 1% Antifoam A (Sigma), then the homogenate was superimposed on a cesium trifluoroacetate (Pharmacia) solution of density 1.64 and subjected to density gradient centrifugation at 44,000 rpm for 14-16 hours at 25°C. After centrifuging, the RNA precipitate was recovered. In this manner, 885 µg of RNA was obtained from 1355 regenerating pancreatic islets (v. Chirgwin, J. M., et al., Biochemistry 18, 5294-5299 (1979)).

### (2) Isolation of poly(A)⁺ RNA

From the RNA obtained in item (1), 17.2 µg of poly(A)⁺ RNA was isolated by oligo-dT cellulose column chromatography (v. H. Aviv and P. Leder, Proc. Natl. Acad. Sci. 69, 1408-1412, 1972).

### (3) Preparation of cDNA library

Using 2 µg of poly(A)⁺ RNA derived from the regenerating pancreatic islets as a template and oligo-dT as a primer, a reaction mediated by 40 units of reverse transcriptase was conducted for 1 hour at 42°C, thereby synthesizing 182 ng of cDNA (first strand). The resulting cDNA-RNA hybrid was treated with 0.75 units of RNase H, 46 units of DNA polymerase I and 4 units of T4 DNA polymerase, thus obtaining 284 ng of double-stranded cDNA. The aforementioned method of cDNA synthesis was essentially that of U. Gubler et al. (Gene 25, 263-269 (1983)).

This double-stranded cDNA was then treated with 15 units of EcoRI methylase (New England Biolabs), and ligated with EcoRI linker (450 ng) by the use of 175 units of T4 DNA ligase (Takara Shuzo), the reaction being carried out for 45 hours at 13°C.

The product of the aforementioned reaction was then digested with 76 units of EcoRI (Takara Shuzo) at 37°C for 2 hours, following which the product was separated into cDNA and digested linkers using Sephacryl S-1000 (Pharmacia). The steps performed thus far yielded 214 ng of EcoRI methylase-treated EcoRI fragments of double-stranded cDNA.

Using 116 units of T4DNA ligase (Takara Shuzo), 20 ng of the aforementioned cDNA was then ligated with 1.23 µg of lambda gt10 arms (dephosphorylated at the 5′ terminus by alkaline phosphatase after EcoRI digestion; Stratagene Co.) at 13°C for 15 hours. The ligate (i.e., lambda gt10-regenerating pancreatic islet cDNA) was packaged using a Gigapack kit (Stratagene Co.) and introduced into the strain Y1089 (DNA cloning, vol. 1, 49-78, 1985, IRL Press), derived from E. coli K12, thereby obtaining 2.8 x 10⁶ transformants.

### III. Differential screening of regenerating pancreatic islet cDNA library: isolation of genes specifically expressed in regenerating pancreatic islets

Phages from 5000 independent plaques randomly selected from the aforementioned regenerating rat pancreatic islet cDNA library prepared in Section II were individually inoculated with toothpicks into NZY-0.2% maltose medium (1% NZ amine, 0.5% Bactoyeast extract, 0.5% sodium chloride and 0.2% maltose) containing the transformed E. coli Y1089 (in such an amount that the OD₆₀₀ of the medium was 0.5) in 96-well microtiter plates and incubated at 37°C overnight to induce phage proliferation.

Next, 2 µl aliquots of each phage solution so obtained were spotted onto nitrocellulose filters (BA85, Schleicher and Schuel) and hybridized with rat insulin II cDNA labelled with ³²P by the nick translation method. By this means, 16% of the clones were identified as insulin cDNA clones, and the non-insulin cDNA clones of the remaining 84% were screened in the following manner.

Regenerating rat pancreatic islet or untreated normal rat pancreatic islet poly(A)⁺ RNA (150 ng) was incubated at 37°C for 2 hours with 15 units of reverse transcriptase (Seikagaku Kogyo) in the presence of 41 ng of oligo-dT₁₂₋₁₈, 20 mM dATP, 20 mM dTTP, 20 mM dGTP, 2 µM dCTP and 60 µCi[α-³²P] dCTP (Amersham). Then, after hydrolyzing the RNA chain with 0.3 N sodium hydroxide at 100°C for 2 minutes, the ³²P labelled single-stranded cDNA so generated was recovered by ethanol precipitation. By this method, probes with a specific activity of 1 x 10⁸ to 2 x 10⁸ cpm per µg poly(A)⁺ RNA were obtained.

Two-microliter aliquots of the phage liquids containing each of the non-insulin cDNA clones were spotted onto two sets of nitrocellulose filters, the filters were treated first with a mixture of 0.5 M sodium hydroxide and 1.5 M sodium chloride, then with a mixture of 0.5 M Tris hydrochloride (pH 8.0) and 1.5 M sodium chloride. Both filters were allowed to stand for 5 minutes at room temperature, then immersed in 2 x SSC (1 x SSC contains 0.15 M sodium chloride and 0.015 M sodium citrate) and then baked at 80°C for 2 hours.

The nitrocellulose filters with the fixed cDNA were then allowed to react for 30 minutes at 65°C in 3 x SSC and at 65°C for 1 hour in 3 x SSC and 1 x FBP (Denhardt's solution, i.e., 0.02% Ficoll 400, 0.02% bovine serum albumin, 0.02% polyvinylpyrrolidone), and then prehybridized for 1 hour at 65°C in a solution containing 1 M sodium chloride, 50 mM Tris hydrochloride (pH 7.4), 10 mM EDTA, 0.1% SDS, 1 x FBP, 10 µg/ml salmon testis DNA and 250 µg/ml poly(U) (Pharmacia). Using cDNA (1.5 x 10⁷ cpm) prepared from regenerating rat pancreatic islet poly(A)⁺ RNA as a probe (supra) for one set of filters and cDNA (1.5 x 10⁷ cpm) prepared from normal pancreatic islet poly(A)⁺ RNA, isolated from untreated rats, as a probe (supra) for the other set of filters, the hybridization reaction in each case was conducted at 65°C for 16 hours. Each hybridization reaction was carried out in a solution containing 1 M sodium chloride, 50 mM Tris hydrochloride (pH 7.4), 10 mM EDTA (pH 8.0), 0.1% SDS, and 250 µg/ml poly(U).

After hybridization, the filters were washed twice for 1-2 minutes with 2 x SSC, then washed four times for 40 minutes in a mixture of 0.1 x SSC and 0.1% SDS, and after drying at 80°C for 1 hour, autoradiography was conducted overnight at -80°C.

By this procedure, a clone specifically hybridizing with regenerating pancreatic islet cDNA was obtained.

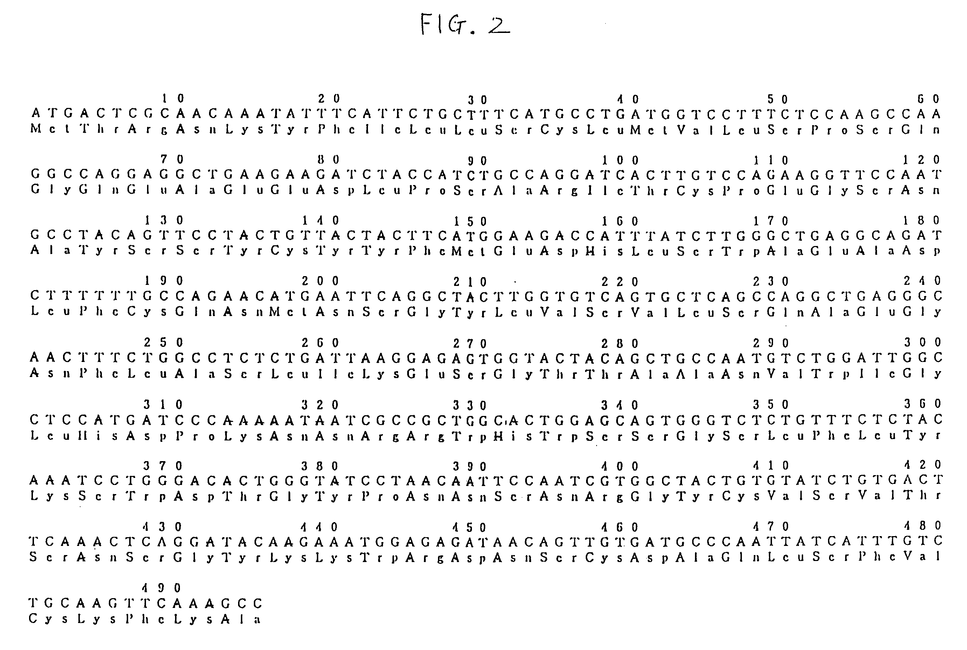

When reproducing the procedures of the present invention, if the probes are constructed with reference to the base sequence shown in Figure 2, then the said procedures are readily implemented.

### IV. Determination of structure of gene specifically expressed in B cells of regenerating pancreatic islets

After purifying the phage DNA obtained from the cDNA clone of the gene specifically expressed in regenerating pancreatic islets obtained in Section III above (v. Molecular Cloning, Cold Spring Harbor Laboratory, New York (1982)), this DNA was digested with EcoRI, and the cDNA insert was separated from the phage arms by 1% agarose gel electrophoresis.

The base sequence of the isolated cDNA was determined by the Sanger technique using M13 (mp18, mp19, Pharmacia) (v. Methods in Enzymology 101, 20-78, 1983).

The base sequencing results revealed that the transcript of the gene specifically expressed in regenerating pancreatic islets, excluding the poly(A) tails, had a total length of 749 nucleotides, encoding a protein composed of 165 amino acid residues, beginning with methionine and terminating with alanine. This base sequence and the corresponding deduced amino acid sequence are shown in Figure 2.

A computer search of the nucleic acid-protein data bases of the European Molecular Biology Laboratory (Heidelberg), GenBank (Cambridge, Massachusetts) and the National Biomedical Research Foundation (Washington, D.C.) was carried out according to the method of Wibur, W. J. and Lipman, D. J., Proc. Natl. Acad. Sci., USA (1983), 80, 726-730. The search revealed that the base sequence of the cDNA cloned as described above, as well as the amino acid sequence deduced from the said base sequence, are different from those of previously known genes or gene products, i.e., no genes or gene products displaying homology of at least 50% with the present genes or gene products were found in the said data searches.

Thus, these extensive data searches indicated that the reg cloned from the cDNA library of regenerating pancreatic islets as described above is an entirely new and hitherto unreported gene.

Using a portion of the base sequence obtained in the above manner, a human reg was isolated from a human pancreatic cDNA library.

### I. Isolation of human reg gene

### 1. Preparation of human pancreatic cDNA library

A) Since the preparation of human regenerating pancreatic islets would be difficult, a cDNA library was prepared from a surgically excised specimen of human pancreas. In rats, reg mRNA is detected in pancreatic tissue.
B) Isolation of RNA
   A human surgically excised pancreas specimen was homogenized with a Polytron in a solution containing 4 M guanidine thiocyanate, 10 mM sodium citrate (pH 7.0), 0.5% sodium lauroylsarcosinate, 0.1 M 2-mercaptoethanol and 1% Antifoam A (Sigma), then the homogenate was superimposed on a cesium trifluoroacetate (Pharmacia) solution of density 1.64 and subjected to density gradient centrifugation at 44,000 rpm for 14-16 hours at 25°C. After centrifuging, the RNA precipitate was recovered. In this manner, 2.526 mg of RNA was obtained from 500 mg of pancreatic tissue (v. Chargwin, J.M., et al., Biochemistry 18, 5294-5299 (1979)).
C) Isolation of poly(A)⁺ RNA
   From the RNA obtained in item b) above, 17.56 µg of poly(A)⁺ RNA was isolated by oligo-dT cellulose column chromatography (v. H. Aviv and P. Leder, Proc. Natl. Acad. Sci. 69, 1408-1412, 1972).
D) Preparation of cDNA library
   Using 1 µg of human pancreatic poly(A)⁺ RNA as a template and oligo-dT as a primer, a reaction mediated by 40 units of reverse transcriptase was conducted for 1 hour at 42°C, thereby synthesizing approximately 100 ng of cDNA (first strand). The resulting cDNA-RNA hybrid was treated with 0.75 units of RNase H, 46 units of DNA polymerase I and 4 units of T4 DNA polymerase, thus obtaining approximately 150 ng of double-stranded cDNA. The aforementioned method of cDNA synthesis was essentially that of U. Gubler et al. (Gene 25, 263-269 (1983)).
   This double-stranded cDNA was then treated with 15 units of EcoRI methylase (New England Biolabs), and ligated with EcoRI linker (450 ng) by a 45-hour reaction at 13°C, mediated by 175 units of T4 DNA ligase (Takara Shuzo).
   The product of the aforementioned reaction was then digested with 76 units of EcoRI (Takara Shuzo) at 37°C for 2 hours, following which the product was separated into cDNA and digested linkers using Sephacryl S-1000 (Pharmacia). The steps performed thus far yielded 17.6 ng of EcoRI methylase-treated EcoRI fragments of double-stranded cDNA.
   Using 116 units of T4DNA ligase (Takara Shuzo), the aforementioned cDNA was then ligated with 1.0 µg of lambda gt10 arms (dephosphorylated at the 5′ terminus by alkaline phosphatase after EcoRI digestion, Stratagene Co.) in a reaction conducted at 13°C for 15 hours. The ligate, lambda gt10-human pancreatic islet cDNA, was packaged using a Gigapack kit (Stratagene Co.) and introduced into the strain Y1089 (DNA cloning, vol. 1, 49-78, 1985, IRL Press), derived from E. coli K12, thereby obtaining a total of 6 x 10⁵ transformants.

### 2. Cloning of human reg cDNA derived from human pancreatic cDNA library

A) The E. coli Y1089 transformants carrying the human cDNA library were added to a maltose medium (0.2% maltose, 1% NZ amine, 0.5% Bactoyeast extract and 0.5% sodium chloride), so that the OD600 of the said medium should be 0.5, and incubated overnight at 37°C, after which 10⁶ plaques were grown on agar medium in dishes of diameter 150 mm and then transferred to nitrocellulose filters.
B) The 60-base oligodeoxyribonucleotide equivalent to the segment containing bases No. 76-135 of the rat reg (see Figure 2) was chemically synthesized using the Applied Biosystems Model 380B DNA synthesizer, and the 5′ terminus of this oligodeoxyribonucleotide was labelled with ³²P using [γ-³²P] ATP and T4 polynucleotide kinase.
C) The filter specimens obtained in item A) above were treated for 5 minutes at room temperature with a mixture of 0.5 N sodium hydroxide and 1.5 M sodium chloride and then for 5 minutes with a mixture of 0.5 M Tris-hydrochloride (pH 8.0) and 1.5 M sodium chloride, then immersed in 2 x SSC (1 x SSC: 0.15 M sodium chloride, 0.015 M sodium citrate) and then baked at 80°C for 2 hours. These filters were then prehybridized at 50°C for 4 hours with a 200 µg/ml solution of E. coli tRNA containing 6 x SSC, 5 x FBP (1 x FBP: 0.02% Ficoll 400, 0.02% bovine serum albumin, 0.02% polyvinylpyrrolidone) and 0.1% SDS, and then hybridized for 12-16 hours at 50°C with a 100 µg/ml E. coli tRNA solution containing 2 ng/ml of ³²P-labelled oligodeoxyribonucleotide, 6 x SSC, 1 x FBP and 0.1% SDS. Then, the filters were washed four times for 30 minutes in 6 x SSC containing 0.1% SDS at 50°C.
D) The results of the above procedure revealed that 58 among the said 10⁶ human pancreatic cDNA clones had hybridized with the 60-base rat reg probe.
E) DNA was isolated from the clone possessing the longest cDNA insert (approximately 900 nucleotides) among the said positive clones, and after digestion with EcoRI, was subcloned in a pBS vector (Stratagene Co.) at the EcoRI site. Since this cDNA possesses an EcoRI cleavage site, the front half and rear half portions of the said DNA were subcloned separately.

### II. Sequencing of human reg cDNA

A) The base sequence of the cDNA subcloned in the PBS vector was determined by the Sanger technique (Methods in Enzymology 101, 20-78, 1983).
B) The sequencing results showed that the human reg cDNA, excluding the poly(A) tail, had a total length 749 nucleotides, encoding a protein composed of 166 amino acid residues, beginning with methionine residue and terminating with asparagine residue.
C) The protein encoded by the human reg was 1 amino acid residue longer than the protein of 165 amino acid residues encoded by the rat reg, and the homology between the coding regions of the base sequences was 75%, while the homology between the corresponding amino acid sequences was 68%.
D) A sequence rich in hydrophobic amino acids is present at the amino terminus region of the human reg protein. This sequence is generally characteristic of the signal peptides seen in secretory proteins. Specifically, the sequence from methionine (-1) up to glutamine (20), glycine (21), glutamine (22), glutamic acid (23), alanine (24), proline (29), glutamine (30) or arginine (32) was presumed to constitute the signal peptide of the human reg protein.

The cDNA obtained in this manner can be expressed by well-known methods. For example, the said cDNA can be introduced into an appropriate expression vector (e.g., pKK223-3, pBS, pDR540, pDR720, pPL-lambda, etc.) under the control of a suitable promoter (e.g., lac, Tac, Trp, P_{L}, etc.), which in turn is introduced into an appropriate host (e.g., E. coli K12 AG-1, MM294, DH1, HB101, C600, etc.) and expressed therein. Subsequent collection and purification are simplified if the reg protein encoded by the reg is secreted out of eukaryotic hosts such as yeast cells, monkey cells, etc. For example, if a yeast host is used, then various well-known expression vectors are applicable, including pGPD-1, pMFα8, AAR6, ABade8, AH5, YEp52, pACF301, pAM82, etc.). If COS monkey cells are used as hosts, then expression vectors such as pKSV-10 are applicable. The host-vector systems suitable for the present purpose are by no means confined to the aforementioned examples, and in fact almost all of the well-known host-vector systems used in genetic engineering appear to be applicable for expression of the present reg protein.

The reg protein expressed in this manner can be purified by conventional procedures using any appropriate combination of techniques such as centrifugation, chromatography, dialysis, salting out, etc.

The amino acid sequence deduced from the human reg contains an abundance of hydrophobic amino acid residues in the vicinity of the amino terminus, these were believed to be the signal peptide, and therefore the aforementioned subsequence was presumed to be the signal sequence. However, in the present invention, entirely contrary to the aforementioned presumption, a reg protein beginning with the glutamine residue at the 20th position from the amino terminus of the amino acid sequence shown in Figure 1 was obtained. Therefore, the present invention provides a new reg protein having the amino acid sequence beginning with the glutamine residue at the 20th position from the amino terminus and terminating with the asparagine residue at the 165 position in Figure 1. Moreover, when this protein is produced by recombinant DNA techniques, then a methionine residue derived from the initiation codon is appended to the amino terminus. Therefore, the present invention also includes the aforementioned protein with an additional methionine residue at the amino terminus.

### Example

### Expression of Human reg Protein in Yeast Microorganisms

E. coli K12 strain (F⁻, hsdR⁺, hsdM⁺, recA⁺, thr, leu, thi, lacY, supE, tonA) was used as the host for the construction of expression vectors.

Saccharomyces cerevisiae AH22 strain (a leu2, his4, can1, cir⁺) (Proc. Natl. Acad. Sci. USA 75, 1929-1933 (1978): Japanese Patent Publication No. 61-55951; this strain has been deposited with the Fermentation Research Institute, Ibaraki, Japan, under the Accession Number FERM BP-312) was used as the host for the expression vector. The yeast cells were cultivated beforehand in YPDA medium (1% yeast extract, 2% polypeptone, 2% glucose and 20 mg/ℓ adenine).

### Medium

Burkholder's minimal medium (Proc. Natl. Acad. Sci. USA, 77, 4504-4508 (1980)) was used for the preparation of Pi-added medium (hereinafter referred to as Pi(+) medium), containing 1.5 g/ℓ potassium dihydrogen phosphate, as well as Pi-deficient medium (hereinafter referred to as Pi(-) medium), containing 1.5 g/ℓ potassium chloride in place of the aforementioned potassium dihydrogen phosphate.

### Enzymes and linkers

Restriction enzymes, T4 DNA ligase and Klenow fragment were purchased from Takara Biochemicals Co., while XhoI linker was purchased from Pharmacia.

### Plasmids

The yeast-E. coli shuttle vector pAM82 (Proc. Natl. Acad. Sci. USA, 80, 1-5 (1983); Japanese Patent Publication No. 61-55951; this strain has been deposited with the Fermentation Research Institute, Ibaraki, Japan, under the Accession Number FERM BP-313) was used as the expression vector. This vector is composed of a 5.5 kb S. cerevisiae DNA fragment containing ARS1, 2 µm ori and leu2, combined with a 3.7 kb fragment of pBR322 containing an ampicillin resistance marker and the replication origin, and in addition a 2.7 kb yeast DNA fragment containing the PHO5 promoter.

The plasmid pGEM4 used in the construction of the expression vector was purchased from Promega Biotec (USA).

An EcoRI site is present in the structural gene of the human reg cDNA, therefore this cDNA was separated into two EcoRI fragments, and each fragment was introduced into the EcoRI site of the pBS vector, respectively.

### Transformation

Transformation was effected in accordance with the method of Kimura et al. (J. Bacteriol. 153, 163-168 (1983)). Leu⁺ strains were selected on Burkholder's minimal medium containing 2% agar.

### Culture and induction

Promotor PHO5 can be regulated by the Pi concentration in the medium (The EMBO Journal, 1, 675-680, 1982). First, the yeast cells were aerobically cultivated in Pi(+) medium fortified with 20 µg/ml of histidine at 30°C for 2 days. To 10 ml of Pi(-) medium fortified with 20 µg/ml of histidine, 200 µl of the above-mentioned culture was transferred and cultivated for 3-5 days in the same manner. From the culture medium, human reg protein was detected.

### Construction of expression vector

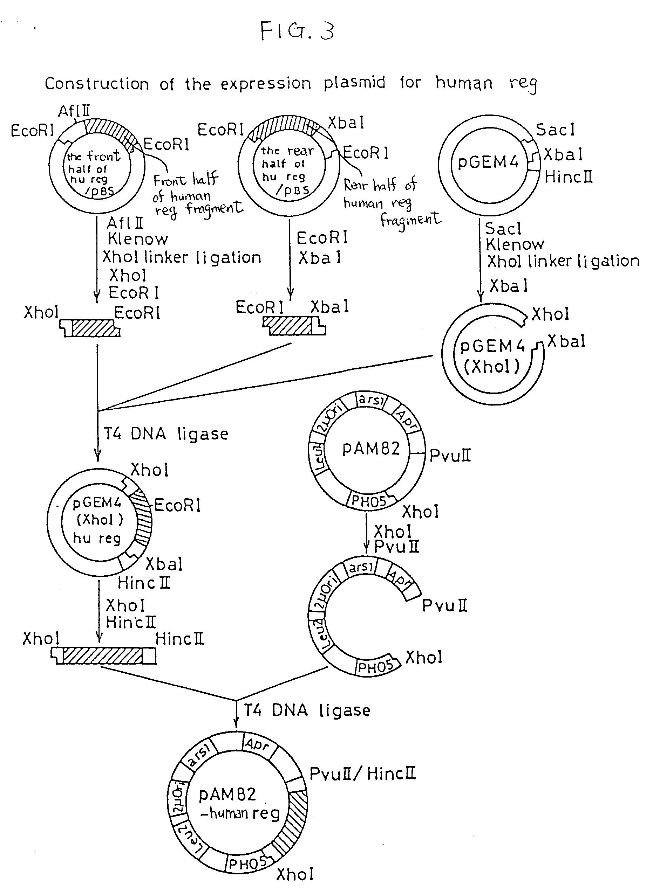

An expression vector was constructed as shown in Figure 3 in accordance with conventional procedures (v. Molecular Cloning, Cold Spring Harbor Laboratory, New York (1982)).

First, the human reg cDNA was divided into two separate fragments (i.e., front half and rear half), and each fragment was inserted into the pBS vector.

The pBS vector with the fragment containing the 5′ terminus region (i.e., the front half of the entire fragment) was digested with AflII and then treated with Klenow fragment, after which XhoI linkers were adjoined and then digested with EcoRI. Concurrently, the pBS vector with the fragment containing the 3′ terminus region (i.e., the rear half of the entire fragment) was digested with EcoRI and XbaI. Using XhoI linker, the SacI site of pGEM4 was converted into an XhoI site, and the aforementioned two human reg cDNA fragments obtained above were inserted into the pGEM4(XhoI). The plasmid pGEM(XhoI)hu reg so assembled was then digested with XhoI and HincII, and the fragment resulting from digestion was finally inserted into pAM82 which had been digested with XhoI and PvuII. The plasmid so constructed was designated pAM82-human reg.

The above-mentioned restriction enzymes were used at a concentration of 3 units/µg DNA, and the corresponding enzymatic reactions were conducted by incubation at 37°C for 1 hour.

### Production of human reg protein

The human reg expression vector constructed by the aforementioned procedure was introduced into E. coli K12 C600, and colonies were selected for ampicillin resistance. Using these transformed strains, a large amount of plasmid DNA was prepared.

The recombinant plasmids which had proliferated in E. coli were then introduced into the yeast host S. cerevisiae AH22 by conventional techniques and leu⁺ colonies were selected.

The transformant strains were inoculated into 500 ml Erlenmeyer flasks containing 100 ml of Pi(+) medium and cultivated for 3 days at 30°C with stirring at 300 rpm using a rotary shaker. Into 5ℓ-minijars containing 2.5 ℓ of Pi(-) medium, 1% of the above-mentioned culture was inoculated, and cultivated for 5 days at 30°C with 500 rpm rotary agitation and ventilation at 1 vvm (2.5 ℓ air/min). Glucose that had been sterilized separately was added to the medium, and 0.4 ml/ℓ of the antifoaming agent polypropylene glycol 2000 was also added. The pH during fermentation was variously adjusted to 5.0, 5.5, 6.0, 6.5, 7.0, 7.5 and 8.0, and including the samples such that the pH was not adjusted, a total of 12.4 ℓ of culture broth was obtained by centrifugation.

### Purification of human reg protein

First, 1 liter of the culture broth containing the reg protein was adjusted to pH 3.4 with 1 N hydrochloric acid, to this solution was added 100 ml of S-Sepharose (fast flow type) equilibrated with 50 mM sodium acetate buffer (pH 3.4) (hereinafter, this buffer is referred to as buffer solution A), and the mixture was gently agitated for 16 hours at 4°C. This S-Sepharose with adsorbed reg protein was packed into a 5 x 8 cm column, washed with buffer solution A containing 0.2 M sodium chloride and eluted with buffer solution A containing 0.5 M sodium chloride. Then, the reg protein fractions corresponding to a molecular weight of approximately 16,000 in SDS-PAGE (15% gel) were pooled, dialyzed against buffer solution A for 16 hours at 4°C, and put on a 0.7 x 25 cm S-Sepharose column equilibrated with buffer solution A. After washing with buffer solution A containing 0.2 M sodium chloride, the adsorbate was eluted with 10 column volumes of buffer solution A under a linear gradient of 0.2 to 0.5 M sodium chloride. The 16 kilodalton reg protein was eluted by buffer solution A containing 0.38 M sodium chloride, and was uniform when the protein was applied to SDS-PAGE (15% gel). After desalinizing by ultrafiltration with an Amicon YM-10 membrane, the purified reg protein was preserved in 10 mM acetic acid at a concentration of 0.1 mg/ml. Approximately 1 mg of reg protein was obtained from 1 liter of yeast culture broth.

### Properties of human reg protein

### Molecular weight

The molecular weight of the purified reg protein obtained by the process described above, as determined by SDS-PAGE, was approximately 16,000.

### Amino acid composition

The reg protein was desalinized by HPLC (Aquapore RP-300 column, Brownlee) and then hydrolyzed by treatment with 4M methanesulphonic acid containing 0.2% 3-(2-aminoethyl)indole at 110°C for 24 hours. Amino acid analysis was performed with a Hitachi Model 835 amino acid analyzer, with the results shown in Table 1.

**Table 1**

| Amino acid | Number of amino acid | |
|---|---|---|
| | Found | Calculated |
| Asp | 18.4 | 17 |
| Thr | 7.1 | 7 |
| Ser | 17.4 | 17 |
| Glu | 14.4 | 15 |
| Pro | 6.1 | 6 |
| Gly | 10.1 | 11 |
| Ala | 8.9 | 8 |
| 1/2Cys | 4.8 | 6 |
| Val | 9.4 | 10 |
| Met | 1.2 | 1 |
| Ile | 4.0 | 4 |
| Leu | 9.1 | 8 |
| Tyr | 7.7 | 7 |
| Phe | 7.4 | 7 |
| Lys | 9.0 | 9 |
| His | 2.5 | 2 |
| Arg | 5.3 | 5 |
| Trp | 6.2 | 6 |
| Total | | 146 |

### Amino terminal sequence

The amino terminal sequence of the reg protein was subjected to analysis by means of an Applied Biosystems 477A protein sequencer, however, the amino acid residue at the amino terminus as well as the following residue were completely undetected. Accordingly, the same analysis was repeated after treatment with pyroglutamate aminopeptidase. As shown in Table 2, 14 of the amino acids in the amino terminus of this enzyme-treated reg protein were determined. The amino terminus of this enzyme-treated protein was a glycine residue, and the amino acid sequence from this glycine residue in position 2 through the serine residue in position 15 agreed with that deduced from the cDNA sequence.

These results indicated that the amino acid at the amino terminus of the reg protein was the glutamine residue at the 20th position of the amino acid sequence shown in Figure 1.

Furthermore, the results of this amino terminal sequence analysis showed that the purified reg protein contains approximately 60% the reg protein beginning with the glutamine residue at the 22nd position of the sequence shown in Figure 1, and approximately 40% of the reg protein of this invention beginning with the glutamine residue at the 20th position of the said sequence.

**Table 2**

| Degradation step | Amino acid residue of reg protein | Recovery (%) |
|---|---|---|
| 1 | (Gln)^{*1} | |
| 2 | Gly | 22.5 |
| 3 | Gln | 17.0 |
| 4 | Glu | 22.8 |
| 5 | Ala | 10.6 |
| 6 | Gln | 21.1 |
| 7 | Thr | 16.4 |
| 8 | Glu | 26.3 |
| 9 | Leu | 34.4 |
| 10 | Pro | 39.3 |
| 11 | Gln | 34.6 |
| 12 | Ala | 20.9 |
| 13 | Arg | 29.0 |
| 14 | Ile | 29.8 |
| 15 | Ser | 96.1 |
| 16 | (Cys)^{*2} | |

| | | |
|---|---|---|
| *1: Unidentified residue. The residue was inferred to be glutamine from results of treatment with pyroglutamate aminopeptidase. | | |
| *2: Unidentified residue. The residue was inferred to be cysteine on the basis of the cDNA sequence. | | |

The human reg of the present invention is believed to be intimately related to the regeneration of insulin-producing pancreatic B cells. Formulation of the reg protein of the present invention, encoded by this gene, in a form suitable for clinical administration would permit radical therapy for diabetes, including the regeneration and activation of the pancreatic B cells of the diabetic patient himself. Thus, the present invention provides a superior therapeutical method to that of the conventional method of symptomatic therapy by administration of insulin.

## Claims (Claims for the following Contracting State(s): DE, GB, FR, IT, NL, SE, LI, CH, BE, AT, LU, DK)

1. A reg protein having an amino acid sequence beginning with the glutamine residue at the 20th position from the amino terminus and terminating with the asparagine residue at the 165th position in Figure 1, or an amino acid sequence containing an additional methionine residue at the amino terminus of said amino acid sequence extending from the glutamine residue to the asparagine residue.

2. A method for producing a reg protein as claimed in claim 1 comprising:
culturing host cells which have an expression vector containing a gene encoding the reg protein of claim 1;
and recovering the reg protein according to claim 1 from the culture.

3. A method according to claim 2, wherein said host is yeast.

## Claims (Claims for the following Contracting State(s): ES, GR)

1. A method for producing a reg protein comprising:
culturing host cells which have an expression vector containing a gene encoding a reg protein having an amino acid sequence beginning with the glutamine residue at the 20th position from the amino terminus and terminating with the asparagine residue at the 165th position in Figure 1, or an amino acid sequence containing an additional methionine residue at the amino terminus of said amino acid sequence extending from the glutamine residue to the asparagine residue;
and recovering said reg protein from the culture.

2. A method according to claim 1, wherein said host is yeast.

## Patentansprüche (Patentansprüche für folgende(n) Vertragsstaat(en): DE, GB, FR, IT, NL, SE, LI, CH, BE, AT, LU, DK)

1. Reg-Protein mit einer Aminosäuresequenz, die unter Bezugnahme auf Figur 1 mit dem Glutaminrest an der Position 20 vom Aminoterminus beginnt und mit dem Asparaginrest an der Position 165 endet, oder mit einer Aminosäuresequenz, die einen zusätzlichen Methioninrest am Aminoterminus der Aminosäuresequenz enthält, die sich vom Glutaminrest bis zum Asparaginrest erstreckt.

2. Verfahren zur Herstellung eines reg-Proteins gemäß Anspruch 1, bei dem man:
- Wirtszellen kultiviert, die über einen Expressionsvektor verfügen, der ein Gen enthält, das für das reg-Protein gemäß Anspruch 1 kodiert,
- das reg-Protein gemäß Anspruch 1 aus der Kultur gewinnt.

3. Verfahren nach Anspruch 2, bei dem der Wirt Hefe ist.

## Patentansprüche (Patentansprüche für folgende(n) Vertragsstaat(en): ES, GR)

1. Verfahren zur Herstellung eines reg-Proteins, bei dem man:
- Wirtszellen kultiviert, die über einen Expressionsvektor verfügen, der ein Gen enthält, das für ein reg-Protein mit einer Aminosäuresequenz kodiert, die unter Bezugnahme auf Figur 1 mit dem Glutaminrest an der Position 20 vom Aminoterminus beginnt und mit dem Asparaginrest an der Position 165 endet, oder mit einer Aminosäuresequenz, die einen zusätzlichen Methioninrest am Aminoterminus der Aminosäuresequenz enthält, die sich von dem Glutaminrest bis zu dem Asparaginrest erstreckt,
- und das reg-Protein aus der Kultur gewinnt.

2. Verfahren nach Anspruch 1, bei dem der Wirt Hefe ist.

## Revendications (Revendications pour l'(les) Etat(s) contractant(s) suivant(s): DE, GB, FR, IT, NL, SE, LI, CH, BE, AT, LU, DK)

1. Protéine reg ayant une séquence en acides aminés commençant par le résidu glutamine à la 20ème position, à partir de l'extrémité amino-terminale, et se terminant par le résidu asparagine à la 165ème position sur la figure 1, ou une séquence en acides aminés contenant un résidu méthionine supplémentaire à l'extrémité aminoterminale de ladite séquence en acides aminés s'étendant à partir du résidu glutamine jusqu'au résidu asparagine.

2. Méthode pour la production d'une protéine reg, selon la revendication 1, comprenant :
la culture de cellules hôtes qui ont un vecteur d'expression contenant un gène codant pour la protéine reg de la revendication 1 ;
et la récupération de la protéine reg, selon la revendication 1, à partir de la culture.

3. Méthode selon la revendication 2, dans laquelle ledit hôte est une levure.

## Revendications (Revendications pour l'(les) Etat(s) contractant(s) suivant(s): ES, GR)

1. Méthode de production de la protéine reg comprenant :
une culture des cellules hôtes qui ont un vecteur d'expression contenant un gène codant pour une protéine reg ayant une séquence en acides aminés commençant par le résidu glutamine à la 20ème position, à partir de l'extrémité amino-terminale, et se terminant par le résidu asparagine à la 165ème position sur la figure 1, ou une séquence en acides aminés contenant un résidu méthionine supplémentaire, à l'extrémité amino-terminale de ladite séquence en acides aminés s'étendant du résidu glutamine jusqu'au résidu asparagine ;
et la récupération de ladite protéine reg à partir de la culture.

2. Méthode selon la revendication 1, dans laquelle ledit hôte est une levure.
